# EUROPEAN PATENT APPLICATION

(11) **EP 4 491 109 A1**
(43) Date of publication of application: **15.01.2025**
(21) Application number: 24185620.2
(22) Date of filing: 01.07.2024
(51) Int. Cl.: A61B 5/091, A61B 5/097, A61M 16/04, A61M 16/08

(54) **DEVICE FOR PERFORMING SPIROMETRY IN PATIENTS WITH TRACHEOSTOMY**

(30) Priority: 12.07.2023 IT 202300014568
(71) Applicant: UNIVERSITA' DEGLI STUDI MAGNA GRAECIA DI CATANZARO, 88100 Catanzaro (IT)
(72) Inventor: ALLEGRA, EUGENIA, 95124 CATANIA (CT) (IT); BIANCO, MARIA RITA, 88050 CROPANI MARINA (CZ) (IT); PULLANO, SALVATORE ANDREA, 88100 CATANZARO (CZ) (IT); OCCHIUZZI, FEDERICO, 87022 CETRARO (CS) (IT); LATELLA, DANIELE, 89124 REGGIO DI CALABRIA (RC) (IT)
(74) Representative: Primiceri, Maria Vittoria

(57) **Abstract**

A device (100) for performing spirometry in patients with tracheostomy comprising a hollow cylindrical body (101), having a first end (101a) provided with a countersink (101aa) for hermetic connection to a disposable spirometer filter, and a second end (101b) provided with a protrusion (101bb) configured to adhere to a connector that adheres to the tracheostoma. Said second end (101b) has a substantially ellipsoidal shaped flange (101bc), suitable for anchoring a cannula-holding collar. Said flange (101bc) comprises two through holes (101c), symmetrically located on opposite sides of the flange (101bc).

## Description

The present invention relates to a device for performing spirometry in patients with tracheostomy.

As it is known, a temporary or permanent tracheostomy surgery is necessary, for example, following laryngeal carcinoma or permanent obstruction of the upper airways, neurological problems or any other condition that does not allow the air coming from outside to reach the lungs through the physiological airways.

A patient undergoing temporary or permanent tracheostomy surgery suffers the alteration of the physiological respiratory tract with functional outcomes that significantly impact the patient's quality of life.

Breathing takes place through a tracheostoma, which is set up during the surgery, consequently, during the inspiratory phase, air from the outside is introduced directly into the trachea and vice versa during the expiratory phase. The exclusion of breathing through the natural route leads to an alteration of the mucociliary clearance of the trachea-bronchial respiratory tract, since the air can no longer be heated, humidified and purified. This condition predisposes the patient to repeated episodes of trachea-bronchial inflammation and to the onset of chronic broncho-pulmonary pathologies with alteration of lung function. Furthermore, it is known that patients suffering from laryngeal carcinoma have mostly been heavy smokers and, therefore, often present, already at diagnosis, with chronic broncho-pulmonary pathologies.

For these reasons, the evaluation of lung function through a spirometry examination should be included among the diagnostic investigations to be performed in oncological follow-ups.

A first solution is described in patent application WO2023042219A1 which reports a respiratory system comprising: an external tube and an internal tube, with dedicated channels for the inhaled and exhaled flows facilitated by valves, so that the inspired air flows through the internal tube and the exhaled air flows through the external tube.

A second solution is reported in patent application ES1278409U which describes a tracheostomy adaptation device, intended to be used in patients with total laryngectomy in respiratory tests. The device comprises: a hollow body equipped with a first tubular sector provided with a proximal end intended to be attached to a peristome of the patient; a second tubular sector connected to the first sector and provided with a distal end intended to be fixed to a functional testing laboratory apparatus; an antibacterial filter housed in the second sector. The device includes a coupling equipped with an adhesive plate with a through hole, designed to fasten the proximal end to the patient.Regarding the proximal end of connection with the patient's tracheostoma, it is noted that the end of the device described does not have any element that guarantees the tightness of the connection and the stability during the execution of a spirometry test. The device disclosed also has a filter which alters the fluid-dynamic resistance of air during the execution of the spirometry.

A third solution is reported in patent application US2010031964A1 which describes a breathing circuit for a tracheostomized patient to provide respiratory assistance and pulmonary function tests. The breathing circuit includes a flow control adapter that directs inhalation and exhalation airflow. The flow control adapter includes a port for coupling to a tracheostomy tube. The flow control adapter also includes a body and one-way valve. There is a one-way valve that prevents the air flowing during inspiration from deviating into the path intended for exhalation.

Also, US2018/078728A1 describes a device aimed to deliver humidified gases to a patient, either through the nose or through the tracheostoma. The use of the device described is therefore substantially different from that of the invention.

However, known devices currently applicable to spirometers do not guarantee a reliable and easy to perform spirometry examination in patients breathing through a tracheostomy.

In fact, known devices do not guarantee an easy and reliable performance of spirometry as causes an alteration of the air flow and do not couple in a stable and hermetic way with the device for performing spirometry.

The aim of the present invention is to provide a device for performing spirometry in patients with tracheostomy, which allows a reliable and repeatable spirometric investigation to be performed on patients with tracheostomy in an easy and reliable manner, therefore having characteristics such as to overcome the limitations of the current known devices for performing spirometry in such patients.

According to the present invention, a device for performing spirometry in patients with tracheostomy is provided, as defined in claim 1.

For a better understanding of the present invention, a preferred embodiment is now described, purely by way of non-limiting example, with reference to the attached figures, in which:

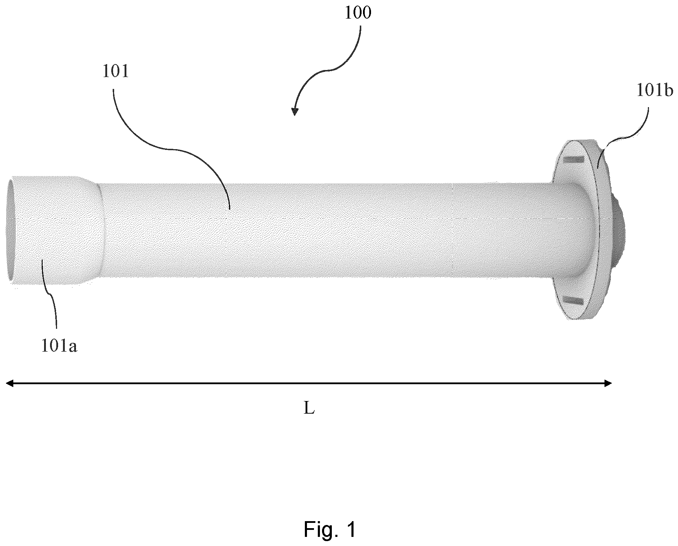

- Figure 1 shows a three-dimensional view of a device for performing spirometry in patients with tracheostomy, according to the invention;

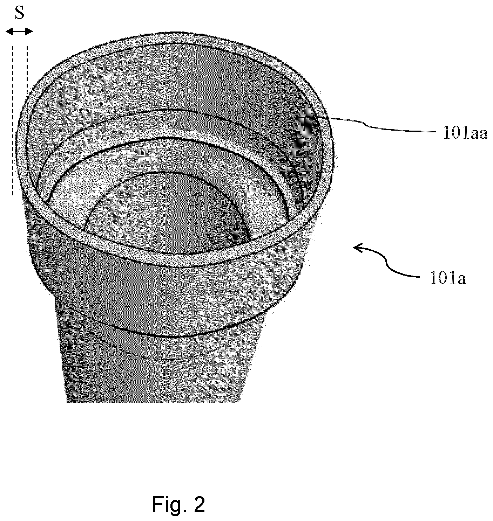

- Figure 2 shows an enlarged three-dimensional view of a first end of the device for performing spirometry in patients with tracheostomy, according to the invention;
- Figure 3 shows an enlarged three-dimensional view of a second end of the device for performing spirometry in patients with tracheostomy, according to the invention;

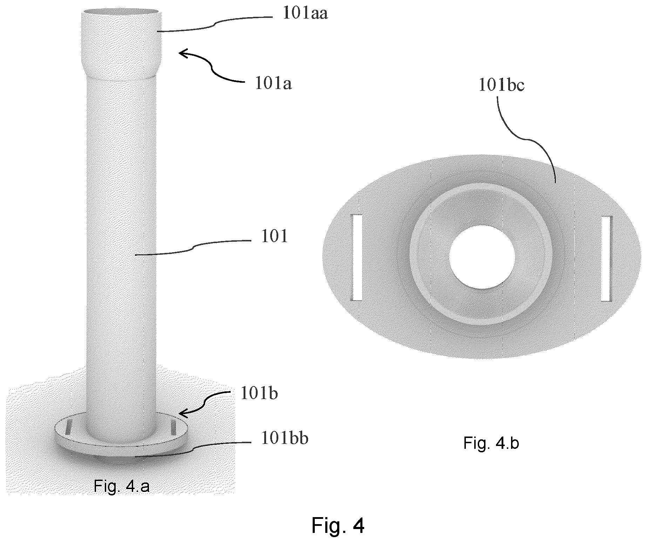

- Figures 4.a and 4.b show respectively a side view and a top view of the device according to the invention.

With reference to these figures, a device for performing spirometry in patients with tracheostomy is shown, according to the invention.

The device 100 for performing spirometry in patients with tracheostomy comprises: a hollow cylindrical body 101 having a first end 101a provided with a countersink 101aa for hermetic connection to a mouthpiece, or disposable spirometer filter, and a second end 101b provided with a protrusion 101bb, preferably circular shaped, configured to adhere to a circular-shaped connector, or adhesive patch, which adheres to the tracheostoma.

According to an aspect of the invention, the second end 101b has a substantially ellipsoidal shaped anchoring flange 101bc suitable for anchoring a cannula-holding collar.

The flange 101bc comprises on its surface two through holes 101c, preferably of a substantially rectangular shape, placed symmetrically on opposite sides of the flange 101bc.

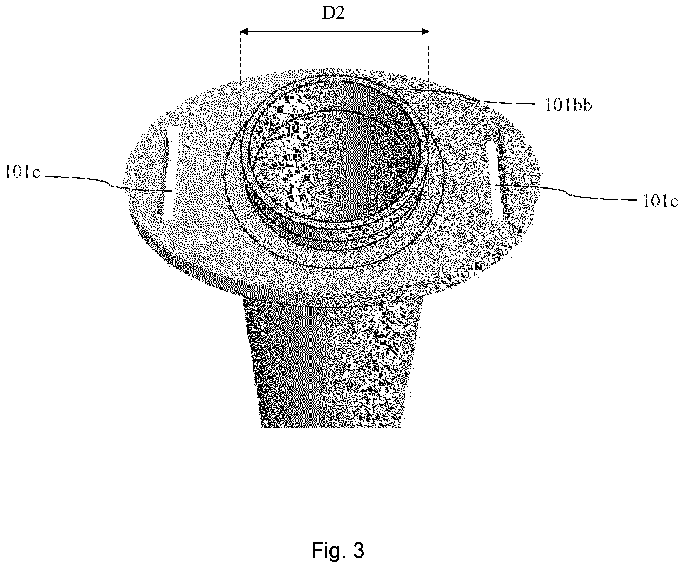

As shown in figure 3, the flange 101bc has a substantially ellipsoidal shape with a central perforation, and the protrusion 101bb protrudes downwards from the central portion of the flange 101bc.

Preferably, the protrusion 101bb has a circular shape.

According to one aspect of the invention, the cylindrical body 101 has a length L between 11 cm and 25 cm, preferably equal to 15 cm, and a thickness S comprised between 0.2 cm and 0.5 cm.

According to one aspect of the invention, the countersinking of the first end 101a has an ellipsoidal shape.

According to an aspect of the invention, the anchoring flange 101bc has a major axis of the ellipse comprised between 5 cm and 8 cm and the minor axis comprised between 3.5 cm and 5 cm.

According to another aspect of the invention, the protrusion 101bb of the second end 101b has an external diameter D2 comprised between 1.7 cm and 2.7 cm (depending on the stomal adhesive), a length L2 comprised between 0.4 cm and 0.7 cm and a thickness S2 between 0.1 cm and 0.3 cm.

According to one aspect of the invention, the device 100 is 3D printable.

Preferably, the device 100 is made of a biocompatible polymeric material modelled by 3D printing or other manufacturing techniques.

According to the present invention, a manufacturing process of the device 100 includes a step of modelling by 3D printing a biocompatible polymer.

Advantageously according to the invention, the device 100 is reusable and compatible with disinfectant solvents.

Therefore, the device for performing spirometry in patients with tracheostomy according to the invention allows it to be adapted to all disposable filters available on the market.

Another advantage of the device for performing spirometry in patients with tracheostomy according to the invention is that it ensures a perfect seal.

Another advantage of the device for performing spirometry in patients with tracheostomy according to the invention is that it is resistant to immersion in solvents based on isopropyl alcohol and 3% hydrogen peroxide.

Also, the device for performing spirometry in patients with tracheostomy according to the invention is robust and reliable.

Finally, it is clear that modifications and variations can be made to the device for performing spirometry in patients with tracheostomy according to the invention here described and illustrated, without departing from the protective scope of the present invention, as defined in the attached claims.

## Claims

1. Device (100) for performing spirometry in patients with tracheostomy comprising a hollow cylindrical body (101), having a first end (101a) provided with a countersink (101aa) for hermetic connection to a disposable spirometer filter, and a second end (101b) provided with a protrusion (101bb) configured to adhere to a connector that adheres to the tracheostoma
**characterized in that** said second end (101b) has a substantially ellipsoidal shaped flange (101bc), suitable for anchoring a cannula-holding collar, and said flange (101bc) comprises two through holes (101c), symmetrically located on opposite sides of the flange (101bc).

2. Device (100) according to claim 1, **characterized in that** the countersink (101aa) has an ellipsoidal shape.

3. Device (100) according to claim 1, **characterized in that** the protrusion (101bb) has a circular shape.

4. Device (100) according to claim 1, **characterized in that** it is made of a 3D printable biocompatible polymeric material.

5. Device (100) according to claim 1, **characterized in that** the cylindrical body (101) has a length L comprised between 11 cm and 25 cm.

6. Device (100) according to claim 1, **characterized in that** the cylindrical body (101) has a thickness S comprised between 0.2 cm and 0.5 cm.

7. Device (100) according to claim 1, **characterized in that** said flange (101bc) has a major axis size comprised between 5 cm and 8 cm and a minor axis size comprised between 3.5 cm and 5 cm.

8. Device (100) according to claim 1, **characterized in that** said protrusion (101bb) of the second end (101b) has an external diameter D2 comprised between 1.7 cm and 2.7 cm, a length L2 comprised between 0.4 cm and 0.7 cm and a thickness S2 comprised between 0.1 cm and 0.3 cm.
